# EUROPÄISCHE PATENTANMELDUNG

(11) **EP 1 941 950 A1**
(43) Veröffentlichungstag der Anmeldung: **09.07.2008**
(21) Anmeldenummer: 06025128.7
(22) Anmeldetag: 05.12.2006
(51) Int. Cl.: B05D 3/10

(54) **Verfahren zur Herstellung poröser Oberflächen auf Metallkomponenten**

(71) Anmelder: Linde Aktiengesellschaft, 65189 Wiesbaden (DE)
(72) Erfinder: Laumen, Christoph, 85356 Freising (DE); Salwén, Anders, Dr., 18262 Djursholm (SE); Wiberg, Sören, 17835 Ekerö (SE)
(74) Vertreter: Gellner, Bernd

(57) **Zusammenfassung**

Die Erfindung betrifft ein Verfahren zur Modifizierung der Oberflächenstruktur eines Metallkörpers, insbesondere zur Erhöhung der Oberflächenporösität, wobei eine Oberfläche des Metallkörpers zunächst einer oxidierenden Atmosphäre und anschließend einer reduzierenden Atmosphäre ausgesetzt wird.

## Beschreibung

Die Erfindung betrifft ein Verfahren zur Modifizierung der Oberflächenstruktur eines Metallkörpers, insbesondere zur Erhöhung der Oberflächenporösität.

Rostfreie austenitische Stähle weisen nach deren Herstellung häufig eine glatte Metalloberfläche ohne Struktur auf und lassen sich daher oft nur schwer benetzen. Dieses Benetzungsverhalten hat großen Einfluss auf die Haftung und Dauerhaftigkeit von Lacken und Beschichtungen, das heißt das Aufbringen von dauerhaften Beschichtungen auf solche Oberflächen gestaltet sich schwierig.

In der Medizintechnik werden biokompatible Werkstoffe, beispielsweise Titan, herangezogen, um daraus Implantate zu fertigen. Auch bei solchen Implantaten bringt eine zu glatte Oberfläche nur schwer lösbare Probleme mit sich. Hierzu zählt beispielsweise ein nur mangelhafter Kontakt zwischen dem Implantat und dem menschlichen Gewebe, in das das Implantat eingesetzt wird.

Aufgabe vorliegender Erfindung ist es daher, ein Verfahren aufzuzeigen, um die Porösität einer Metalloberfläche zu erhöhen.

Diese Aufgabe wird durch ein Verfahren zur Modifizierung der Oberflächenstruktur eines Metallkörpers, insbesondere zur Erhöhung der Oberflächenporösität, gelöst, welches sich dadurch auszeichnet, dass eine Oberfläche des Metallkörpers zunächst einer oxidierenden Atmosphäre und anschließend einer reduzierenden Atmosphäre ausgesetzt wird.

Erfindungsgemäß wird die Metalloberfläche zunächst oxidiert und anschließend reduziert. Während des Oxidationsschrittes bildet sich auf der Metalloberfläche in bekannter Weise eine Oxidschicht aus. In einem zweiten Schritt wird nun die oxidierte Metalloberfläche einer reduzierenden Atmosphäre ausgesetzt. Bei dieser Behandlung wird zumindest ein Teil der vorhandenen Oxide durch Reduktion entfernt, wobei entsprechende Poren in der Oberfläche zurückbleiben.

Durch die erfindungsgemäße Durchführung einer Oxidation gefolgt von einer Reduktion der Metalloberfläche entsteht so eine poröse Oberflächenstruktur, die sich gut benetzen lässt.

Von Vorteil wird das Verfahren genutzt, um die Oberfläche eines Körpers aus rostfreiem austenitischem Stahl, einer Co-Cr-Legierung, Titan, Tantal oder einer diese Stoffe enthaltenden Legierung erfindungsgemäß zu behandeln. Hierbei wird entweder der ganze Körper oder nur die zu behandelnde Oberfläche des Körpers zunächst einer oxidierenden und anschließend einer reduzierenden Atmosphäre ausgesetzt.

Die Art und Ausprägung der erzeugten Oberflächenporösität hängt von den Verfahrensparametern des Oxidationsschrittes und des Reduktionsschrittes ab. Um eine den gestellten Anforderungen optimal angepasste Porenstruktur zu erhalten, müssen die Parameter, insbesondere Temperatur und Dauer, der Oxidations- und der Reduktionsphase geeignet gewählt werden.

Diese Parameter hängen von der Art des Metalls beziehungsweise der Legierung ab, deren Oberfläche modifiziert werden soll, von der Art des eingesetzten Oxidations- und Reduktionsmittels und von der gewünschten Porenstruktur, beispielsweise deren Größe. Vorzugsweise werden deshalb die Temperatur der Oxidationsatmosphäre und die der Reduktionsatmosphäre sowie die jeweiligen Einwirkungszeiten auf die zu behandelnde Oberfläche in Abhängigkeit von der Art des Metalls beziehungsweise der Legierung ab, in Abhängigkeit von der Art des eingesetzten Oxidations- und Reduktionsmittels und / oder in Abhängigkeit von der gewünschten Porenstruktur eingestellt.

Es hat sich gezeigt, dass bei der Behandlung von Stählen die Oxidation bevorzugt bei einer Temperatur zwischen 1000°C und 1300 °C, besonders bevorzugt zwischen 1100 °C und 1200 °C, durchgeführt wird. Für die Dauer der Wärmebehandlung wird vorzugsweise eine Zeitspanne zwischen 10 und 200 Minuten, vorzugsweise zwischen 30 und 120 Minuten, gewählt. Für Titan, Cr-Co oder andere Werkstoffe können die genannten Parameter als erster Anhaltspunkt dienen, in der Regel ist aber eine weitere Optimierung sinnvoll.

Der Reduktionsschritt wird bevorzugt bei Temperaturen zwischen 1200 °C und 1300 °C für die Dauer von 5 Minuten bis 120 Minuten durchgeführt.

Zur Erzeugung der Oxidschicht wird die Oberfläche von Vorteil einer Atmosphäre mit einem Sauerstoffanteil von 1 bis 100% ausgesetzt. Vorzugsweise wird Luft als Oxidationsmittel verwendet. Je nach zu oxidierendem Metall und der gewünschten Ausprägung der Porenstruktur kann es aber auch günstig sein, die Oxidation mit Hilfe von mit Sauerstoff angereicherter Luft oder technisch reinem Sauerstoff als Oxidationsmittel durchzuführen. Bevorzugt wird hierbei eine Atmosphäre mit einem Sauerstoffgehalt von mindestens 50%, besonders bevorzugt mindestens 90% Sauerstoff verwendet. Gegebenenfalls ist in diesem Fall die Temperatur der Oxidationsbehandlung geeignet anzupassen. Weiter hat sich gezeigt, dass auch andere Oxidationsmittel, wie zum Beispiel feuchte Luft, Wasserdampf, Kohlendioxid oder Gemische aus Stickstoff und Sauerstoff geeignet sind, um die erfindungsgemäße Oxidation hervorzurufen.

Die Oxidation der Oberfläche kann ebenso als Nebenwirkung in einem anderen Wärmebehandlungs- oder Umformungsschritt entstehen beziehungsweise erzeugt werden. So wird beispielsweise beim Warmwalzen die Oberfläche des Metalls bereits oxidiert, so dass eine weitere separate Oxidation nicht unbedingt durchgeführt werden muss.

Die Reduktion der Oxidschicht wird vorzugsweise in einer Wasserstoffatmosphäre durchgeführt. Es hat sich dabei bewährt, eine reduzierende Atmosphäre mit einem Wasserstoffgehalt von mindestens 75%, bevorzugt mindestens 90 %, besonders bevorzugt mindestens 98% Wasserstoff einzusetzen. Anstelle oder zusätzlich zu Wasserstoff kann auch eine CO-haltige Atmosphäre zur Reduktion verwendet werden.

In einer bevorzugten Ausführungsform der Erfindung wird die Oxidation in Luft und die anschließnede Reduktion in einer Atmosphäre reinen Wasserstoffs durchgeführt.

Ziel des erfindungsgemäßen Verfahrens ist es, eine poröse Metalloberfläche zu schaffen. Hierzu werden während des Oxidationsschrittes zunächst Oxide auf der Oberfläche aufgebaut und während des Reduktionsschrittes wieder entfernt, so dass die gewünschten Poren verbleiben. Von Vorteil werden diese beiden Schritte, nämlich die Oxidation und die Reduktion der Metalloberfläche, unmittelbar aufeinanderfolgend durchgeführt. Insbesondere ist es von Vorteil, wenn die Oberfläche zwischen dem Oxidations- und dem Reduktionsschritt keinem anderen Wärmebehandlungsprozess ausgesetzt wird.

Die Erfindung wird bevorzugt zur Modifizierung der Oberflächenstruktur von Vorrichtungen für medizinische oder pharmazeutische Zwecke genutzt. So können beispielsweise medizinische Implantate, z.B. Zahnimplantate, ärztliche Instrumente, Prothesen oder künstlichen Gelenke, oder Werkstoffe, aus denen solche Implantate und Prothesen gefertigt werden, erfindungsgemäß modifiziert werden.

Die Oberflächenporösität verbessert den Kontakt zwischen dem Implantat und dem menschlichen oder tierischen Gewebe oder Knochen. Andererseits werden die Implantate oder Prothesen, aber auch ärztliche Instrumente, häufig mit Beschichtungen, zum Beispiel einer Hydroxiapatitbeschichtung, versehen. Durch den Einsatz des erfindungsgemäßen Verfahrens wird eine deutlich verbesserte Haftung derartiger Schichten erzielt.

Insbesondere im Bereich der Medizintechnik und Chirurgie, aber auch in anderen Bereichen der Technik, können die erfindungsgemäß gebildeten Poren dazu genutzt werden, um darin Wirkstoffe, Isotope oder Pharmazeutika einzulagern, die an die Umgebung abgegeben oder in das umgebende Gewebe eingebracht werden sollen.

Ein weiteres bevorzugtes Einsatzgebiet des erfindungsgemäßen Verfahrens ist die Behandlung von metallischen Oberflächen, um deren Haftungseigenschaften für eine anschließende Lackierung oder Beschichtung zu verbessern.

Bisher werden Oberflächen, die eine definierte Struktur oder Porösität haben sollen, häufig durch Pulverbeschichtung oder Aufsintem von Pulver hergestellt. Die vorliegende Erfindung stellt eine kosteneffektive Möglichkeit dar, diese relativ aufwändigen Verfahren zu ersetzen.

Die Erfindung hat zahlreiche Vorteile gegenüber dem Stand der Technik. Mit dem erfindungsgemäßen Verfahren kann die Oberflächenporösität maßgeschneidert werden. Tiefe und Größe der Poren können durch geeignete Wahl der Verfahrensparameter im Oxidations- und im Reduktionsschritt eingestellt werden. Insbesondere rostfreie austenitische Stähle, Co-Cr-Legierungen, Titan- und Tantalwerkstoffe, die häufig eine glatte Oberflächenstruktur besitzen, können erfindungsgemäß vorbereitet werden, damit nachfolgende Beschichtungen besser und dauerhaft halten.

Die Erfindung sowie weitere Einzelheiten der Erfindung werden im Folgenden anhand von in den Zeichnungen dargestellten Ausführungsbeispielen näher erläutert. Hierbei zeigen die Figuren Aufnahmen von erfindungsgemäß behandelten Metalloberflächen.

### Beispiel 1:

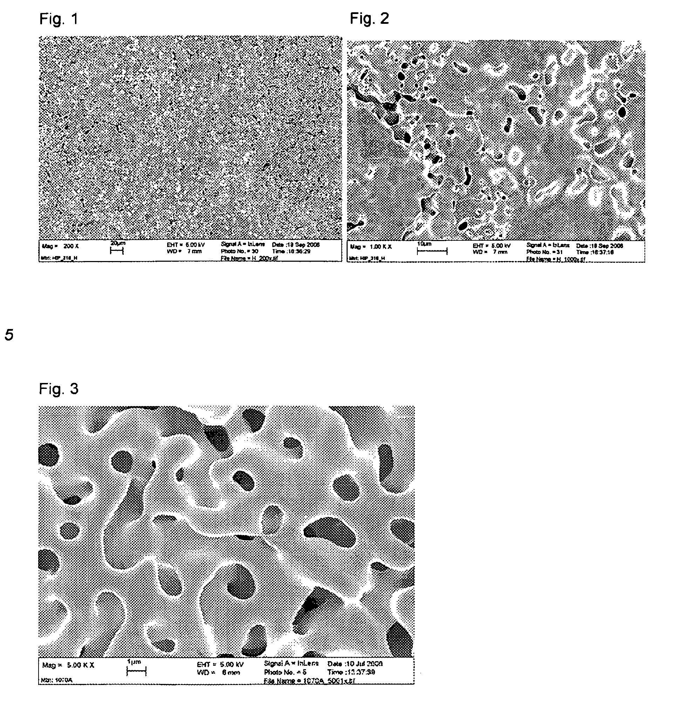

Ein Stahl des Typs AISI316 wurde bei 1200 °C 30 Minuten in einer Sauerstoffatmosphäre oxidiert und nachfolgend bei 1150°C ebenfalls 30 Minuten in einer 100%-Wasserstoff-Atmosphäre reduziert. Dabei entstanden Poren mit einer Größe zwischen 1 Mikrometer und 10 Mikrometer und die sich ausbildenden Porenkanäle erreichten eine Tiefe von mehreren Mikrometern. Die Figuren 1 und 2 zeigen Aufnahmen der erzeugten Poren.

### Beispiel 2:

Figur 3 zeigt die vergrößerte Darstellung eines warmgewalzten Drahtes, dessen Oberfläche während des Warmwalzens oxidiert ist und der anschließend bei 1170°C in einer Wasserstoffatmosphäre reduziert wurde. Die Porösität der Oberfläche ist deutlich zu erkennen.

### Beispiel 3:

Die Oberflächenporösität von Zahnimplantaten aus Titan wurde erfindungsgemäß modifiziert. Es wurde festgestellt, dass die Oberflächentopographie der Zahnimplantate einen wesentlichen Einfluss auf den Ablauf und die Schnelligkeit biologischer Prozesse nach dem Einsetzen in den menschlichen oder tierischen Körper hat. Dies gilt für Vorgänge im Nanometerbereich bis hin zu Prozessen auf der Makroebene bzw. mit Makroteilchen.

## Patentansprüche

1. Verfahren zur Modifizierung der Oberflächenstruktur eines Metallkörpers, insbesondere zur Erhöhung der Oberflächenporösität, **dadurch gekennzeichnet, dass** eine Oberfläche des Metallkörpers zunächst einer oxidierenden Atmosphäre und anschließend einer reduzierenden Atmosphäre ausgesetzt wird.

2. Verfahren nach Anspruch 1, **dadurch gekennzeichnet, dass** die Oberfläche eines Körpers aus rostfreiem austenitischem Stahl, einer Co-Cr-Legierung, Titan, Tantal oder einer diese Stoffe enthaltenden Legierung wärmebehandelt wird.

3. Verfahren nach einem der Ansprüche 1 oder 2, **dadurch gekennzeichnet, dass** die Oberfläche bei einer Temperatur zwischen 1000 °C und 1300 °C, vorzugsweise zwischen 1100 °C und 1200 °C, der oxidierenden Atmosphäre ausgesetzt wird.

4. Verfahren nach einem der Ansprüche 1 bis 3, **dadurch gekennzeichnet, dass** die oxidierende Atmosphäre einen Sauerstoffgehalt von mindestens 50%, bevorzugt mindestens 75%, besonders bevorzugt mindestens 90%, aufweist.

5. Verfahren nach einem der Ansprüche 1 bis 4, **dadurch gekennzeichnet, dass** die Oberfläche für eine Zeitdauer zwischen 10 und 200 Minuten, vorzugsweise zwischen 30 und 120 Minuten, der oxidierenden Atmosphäre ausgesetzt wird.

6. Verfahren nach einem der Ansprüche 1 bis 5, **dadurch gekennzeichnet, dass** die Oberfläche bei einer Temperatur zwischen 1200 °C und 1300 °C der reduzierenden Atmosphäre ausgesetzt wird.

7. Verfahren nach einem der Ansprüche 1 bis 6, **dadurch gekennzeichnet, dass** die reduzierende Atmosphäre einen Wasserstoffgehalt von mindestens 75%, bevorzugt mindestens 90 %, besonders bevorzugt mindestens 99%, aufweist.

8. Verfahren nach einem der Ansprüche 1 bis 7, **dadurch gekennzeichnet, dass** Luft als oxidierende Atmosphäre und reiner Wasserstoff als reduzierende Atmosphäre eingesetzt werden.

9. Verfahren nach einem der Ansprüche 1 bis 8, **dadurch gekennzeichnet, dass** die Oberflächenstruktur von Vorrichtungen für medizinische oder pharmazeutische Zwecke, insbesondere von Implantaten, modifiziert wird.

10. Verfahren nach einem der Ansprüche 1 bis 9, **dadurch gekennzeichnet, dass** die Porösität der Oberfläche erhöht und eine aktive Substanz, insbesondere ein medizinisch oder pharmazeutisch wirksamer Wirkstoff, in die Poren eingebracht wird.
